# EUROPEAN PATENT SPECIFICATION

(11) **EP 1 483 387 B1**
(45) Date of publication and mention of the grant of the patent: **02.07.2008**
(21) Application number: 03708152.8
(22) Date of filing: 27.02.2003
(51) Int. Cl.: C12N 15/31, C12P 13/08, C12P 13/04, C07K 14/245

(54) **PROCESS FOR THE PREPARATION OF L-THREONINE USING STRAINS OF THE FAMILY ENTEROBACTERIACEAE**
VERFAHREN ZUR HERSTELLUNG VON L-THREONIN UNTER VERWENDUNG VON STÄMMEN DER ENTEROBACTERIACEAE FAMILIE
PROCEDE DE PREPARATION DE L-THREONINE AU MOYEN DE SOUCHES DE LA FAMILLE ENTEROBACTERIACEAE

(30) Priority: 13.03.2002 DE 10210966; 21.03.2002 US 365829 P
(43) Date of publication of application: 08.12.2004
(73) Proprietor: Evonik Degussa GmbH, 40474 Düsseldorf (DE)
(72) Inventor: RIEPING, Mechthild, 33619 Bielefeld (DE); HERMANN, Thomas, 33739 Bielefeld (DE); FARWICK, Mike, 45131 Essen (DE)
(86) International application number: PCT/EP2003/001997
(87) International publication number: WO 2003/076627

(56) References cited:
- EP-A- 0 643 135
- EP-A- 0 994 190
- WO-A-99/53035
- US-A- 4 278 765
- NOTLEY-MCROBB L ET AL.: "Adaptive mgl-regulatory mutations and genetic diversity evolving in glucose-limited Escherichia coli populations" ENVIRONMENTAL MICROBIOLOGY, vol. 1, no. 1, February 1999 (1999-02), pages 33-43, XP001147506 ISSN: 1462-2912
- ALEXANDER D M ET AL.: "Carbohydrate uptake genes in Escherichia coli are induced by carbon starvation." CURRENT MICROBIOLOGY, vol. 27, no. 6, 1993, pages 335-340, XP008018907 ISSN: 0343-8651
- HOGG R W ET AL.: "Nucleotide sequence and analysis of the mgl operon of Escherichia coli K12." MOLECULAR & GENERAL GENETICS, vol. 229, no. 3, October 1991 (1991-10), pages 453-459, XP001149195 ISSN: 0026-8925 cited in the application
- MÜLLER N ET AL.: "Cloning of mglB, the structural gene for the galactose-binding protein of Salmonella typhimurium and Escherichia coli." MOLECULAR & GENERAL GENETICS, vol. 185, no. 3, 1982, pages 473-480, XP008018906 ISSN: 0026-8925
- HARAYAMA S ET AL.: "Characterization of the mgl operon of Escherichia coli by transposon mutagenesis and molecular cloning." JOURNAL OF BACTERIOLOGY, vol. 153, no. 1, January 1983 (1983-01), pages 408-415, XP008018905 ISSN: 0021-9193 cited in the application
- MICHAL G: "Biochemical pathways: an atlas of biochemistry and molecular biology" 1999 , JOHN WILEY & SONS INC. AND SPEKTRUM AKADEMISCHER VERLAG , NEW YORK - HEIDELBERG XP002242199 ISBN: 0-471-33130-9 figure 3.8-2 figures 4.2-1, 4.5-1 and 4.5-2
- KRAEMER R: "Genetic and physiological approaches for the production of amino acids" JOURNAL OF BIOTECHNOLOGY, vol. 45, no. 1, 1996, pages 1-21, XP002178648 ISSN: 0168-1656
- JETTEN M S M ET AL.: "Recent advances in the physiology and genetics of amino acid-producing bacteria." CRC CRITICAL REVIEWS IN BIOTECHNOLOGY, vol. 15, no. 1, 1995, pages 73-103, XP000613291 ISSN: 0738-8551

### Sequence Listing

<110> Degussa AG
<120> Process for the preparation of L-amino acids using strains of the family Enterobacteriaceae
<130> 020113 BT
<160> 2
<170> PatentIn version 3.1
<210> 1
   <211> 29
   <212> DNA
   <213> Artificial sequence
<220>
   <221> Primer
   <222> (1)..(29)
   <223> mglB1
<400> 1
   cctgctctag ataaaccgga gataccatg 29
<210> 2
   <211> 30
   <212> DNA
   <213> Artificial sequence
<220>
   <221> Primer
   <222> (1)..(30)
   <223> mglB2
<400> 2
   ccgaagcttg ttggccatac aataagggcg 30

## Description

The present invention relates to a fermentation process for the preparation of L-threonine, using strains of the family Enterobacteriaceae in which the mglB gene is overexpressed.

### State of the art

L-Amino acids, especially L-threonine, are used in human medicine and in the pharmaceutical industry, in the food industry and very particularly in animal nutrition.

It is known to prepare L-amino acids by the fermentation of strains of Enterobacteriaceae, especially Escherichia coli (E. coli) and Serratia marcescens. Because of their great importance, attempts are constantly being made to improve the preparative processes. Improvements to the processes may relate to measures involving the fermentation technology, e.g. stirring and oxygen supply, or the composition of the nutrient media, e.g. the sugar concentration during fermentation, or the work-up to the product form, e.g. by ion exchange chromatography, or the intrinsic productivity characteristics of the microorganism itself.

The productivity characteristics of these microorganisms are improved by using methods of mutagenesis, selection and mutant choice to give strains which are resistant to antimetabolites, e.g. the threonine analog α-amino-β-hydroxyvaleric acid (AHV), or auxotrophic for metabolites of regulatory significance, and produce L-amino acids, e.g. L-threonine.

Methods of recombinant DNA technology have also been used for some years to improve L-amino acid-producing strains of the family Enterobacteriaceae by amplifying individual amino acid biosynthesis genes and studying the effect on production.

### Object of the invention

The object which the inventors set themselves was to provide novel procedures for improving the preparation of L-threonine by fermentation.

### Description of the invention

The invention provides a fermentation process for the preparation of L-threonine using microorganisms of the family Enterobacteriaceae which, in particular, already produce L-amino acids and in which the nucleotide sequence coding for the mglB gene is overexpressed.

The term "L-amino acids" or "amino acids" mentioned hereafter is to be understood as meaning L-threonine including its salts.

In this context the term "overexpression" describes the increase, in a microorganism, of the intracellular activity of one or more enzymes or proteins coded for by the appropriate DNA, for example by increasing the copy number of the gene or genes, using a strong promoter or a gene or allele coding for an appropriate enzyme or protein with a high activity, and optionally combining these measures.

Through the measures of overexpression, the activity or concentration of the appropriate protein is generally increased at least by 10%, 25%, 50%, 75%, 100%, 150%, 200%, 300%, 400% or 500%, and at most by up to 1000% or 2000%, based on that of the wild-type protein or the activity or concentration of the protein in the starting microorganism.

The process comprises carrying out the following steps:
a) fermentation of microorganisms of the family Enterobacteriaceae which produce L-threonine and in which the mglB gene, from Enterobacteriaceae, or nucleotide sequences coding therefore, is (are) overexpressed compared to the unmodified microorganisms,
b) enrichment of said L-amino acid in the medium or in the cells of the microorganisms, and
c) isolation of the desired L-amino acid, where constituents of the fermentation broth, and/or all or part (>0 to 100) of the biomass, optionally remain in the product.

The microorganisms provided by the present invention can produce L-threonine from glucose, sucrose, lactose, fructose, maltose, molasses, optionally starch or optionally cellulose, or from glycerol and ethanol. Said microorganisms are representatives of the family Enterobacteriaceae selected from the genera Escherichia, Erwinia, Providencia and Serratia. The genera Escherichia and Serratia are preferred. The species Escherichia coli and Serratia marcescens may be mentioned in particular among the genera Escherichia and Serratia respectively. Examples of suitable strains, particularly L-threonine-producing strains, of the genus Escherichia, and especially of the species Escherichia coli, are:
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132

Examples of suitable L-threonine-producing strains of the genus Serratia, and especially of the species Serratia marcescens, are:
Serratia marcescens HNr21
Serratia marcescens TLr156
Serratia marcescens T2000

L-Threonine-producing strains of the family Enterobacteriaceae preferably possess, inter alia, one or more genetic or phenotypic characteristics selected from the group comprising resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidine, resistance to rifampicin, resistance to valine analogs such as valine hydroxamate, resistance to purine analogs such as 6-dimethylaminopurine, need for L-methionine, optionally partial and compensatable need for L-isoleucine, need for meso-diaminopimelic acid, auxotrophy in respect of threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally capability for sucrose utilization, amplification of the threonine operon, amplification of homoserine dehydrogenase I-aspartate kinase I, preferably of the feedback-resistant form, amplification of homoserine kinase, amplification of threonine synthase, amplification of aspartate kinase, optionally of the feedback-resistant form, amplification of aspartate semialdehyde dehydrogenase, amplification of phosphoenolpyruvate carboxylase, optionally of the feedback-resistant form, amplification of phosphoenolpyruvate synthase, amplification of transhydrogenase, amplification of the RhtB gene product, amplification of the RhtC gene product, amplification of the YfiK gene product, amplification of a pyruvate carboxylase and attenuation of acetic acid formation.

It has been found that the production of L-threonine by microorganisms of the family Enterobacteriaceae is improved after overexpression of the mglB gene.

The nucleotide sequences of the genes of Escherichia coli belong to the state of the art (cf. literature references below) and can also be taken from the genome sequence of Escherichia coli published by Blattner et al. (Science 277, 1453-1462 (1997)).

The mglB gene is described inter alia by the following data:

| | |
|---|---|
| Name: | periplasmatic galactose-binding transport protein, receptor for galactose chemotaxis |
| Reference: | Hogg et al.; Molecular and General Genetics 229(3), 453-459 (1991) Muller et al.; Molecular and General Genetics 185(3), 473-480 (1982) |
| Accession no.: | AE000304 |

The nucleic acid sequences can be taken from the data banks of the National Center for Biotechnology Information (NCBI) of the National Library of Medicine (Bethesda, MD, USA), the nucleotide sequence data bank of the European Molecular Biologies Laboratories (EMBL, Heidelberg, Germany, or Cambridge, UK) or the DNA Databank of Japan (DDBJ, Mishima, Japan).

The genes described in the literature references cited can be used according to the invention. It is also possible to use alleles of the genes which result from the degeneracy of the genetic code or from neutral sense mutations. The use of endogenous genes is preferred.

The term "endogenous genes" or "endogenous nucleotide sequences" is to be understood as meaning the genes or alleles, or nucleotide sequences, present in the population of a species.

Overexpression can be achieved for example by increasing the expression of the genes or enhancing the catalytic properties of the proteins. Both measures may optionally be combined.

The concentration of the transport protein coded for by the polynucleotide mglB can be determined by the method described by Richarme and Caldas (Journal of Biological Chemistry 272(25), 15607-15612 (1997)).
Overexpression can be achieved by increasing the copy number of the appropriate genes or mutating the promoter and regulatory region or the ribosome binding site located upstream from the structural gene. Expression cassettes incorporated upstream from the structural gene work in the same way. Inducible promoters additionally make it possible to increase expression in the course of L-threonine production by fermentation. Measures for prolonging the life of the mRNA also improve expression. Furthermore, the enzyme activity is also enhanced by preventing the degradation of the enzyme protein. The genes or gene constructs can either be located in plasmids of variable copy number or be integrated and amplified in the chromosome. Alternatively, it is also possible to achieve overexpression of the genes in question by changing the composition of the media and the culture technique.

Those skilled in the art will find relevant instructions inter alia in Chang and Cohen (Journal of Bacteriology 134, 1141-1156 (1978)), Hartley and Gregori (Gene 13, 347-353 (1981)), Amann and Brosius (Gene 40, 183-190 (1985)), de Broer et al. (Proceedings of the National Academy of Sciences of the United States of America 80, 21-25 (1983)), LaVallie et al. (BIO/TECHNOLOGY 11, 187-193 (1993)), PCT/US97/13359, Llosa et al. (Plasmid 26, 222-224 (1991)), Quandt and Klipp (Gene 80, 161-169 (1989)), Hamilton et al. (Journal of Bacteriology 171, 4617-4622 (1989)), Jensen and Hammer (Biotechnology and Bioengineering 58, 191-195 (1998)) and well-known textbooks on genetics and molecular biology.

Plasmid vectors replicatable in Enterobacteriaceae, e.g. cloning vectors derived from pACYC184 (Bartolomé et al.; Gene 102, 75-78 (1991)), pTrc99A (Amann et al.; Gene 69, 301-315 (1988)) or pSC101 derivatives (Vocke and Bastia; Proceedings of the National Academy of Sciences USA 80(21), 6557-6561 (1983)), can be used. In one process according to the invention, it is possible to use a strain transformed with a plasmid vector, said plasmid vector carrying at least one nucleotide sequence coding for the mglB gene.

Also, mutations which affect the expression of the appropriate genes can be transferred to different strains by sequence exchange (Hamilton et al.; Journal of Bacteriology 171, 4617-4622 (1989)), conjugation or transduction.

Furthermore, for the production of L-threonine with strains of the family Enterobacteriaceae, it can be advantageous not only to overexpress the mglB gene but also to overexpress one or more enzymes of the known threonine biosynthetic pathway, or enzymes of the anaplerotic metabolism, or enzymes for the production of reduced nicotinamide adenine dinucleotide phosphate, or glycolytic enzymes, or PTS enzymes or enzymes of sulfur metabolism. The use of endogenous genes is generally preferred.

Thus, for example, one or more genes selected from the group comprising:
- the thrABC operon coding for aspartate kinase, homoserine dehydrogenase, homoserine kinase and threonine synthase (US-A-4,278,765),
- the pyc gene coding for pyruvate carboxylase (DE-A-19 831 609),
- the pps gene coding for phosphoenolpyruvate synthase (Molecular and General Genetics 231(2), 332-336 (1992)),
- the ppc gene coding for phosphoenolpyruvate carboxylase (Gene 31, 279-283 (1984)),
- the pntA and pntB genes coding for transhydrogenase (European Journal of Biochemistry 158, 647-653 (1986)),
- the rhtB gene for homoserine resistance (EP-A-0 994 190),
- the mqo gene coding for malate:quinone oxidoreductase (DE 100 348 33.5),
- the rhtC gene for threonine resistance (EP-A-1 013 765),
- the thrE gene of Corynebacterium glutamicum coding for threonine export protein (DE 100 264 94.8),
- the gdhA gene coding for glutamate dehydrogenase (Nucleic Acids Research 11, 5257-5266 (1983); Gene 23, 199-209 (1983)),
- the hns gene coding for DNA binding protein HLP-II (Molecular and General Genetics 212, 199-202 (1988)),
- the pgm gene coding for phosphoglucomutase (Journal of Bacteriology 176, 5847-5851 (1994)),
- the fba gene coding for fructose biphosphate aldolase (Biochemical Journal 257, 529-534 (1989)),
- the ptsH gene of the ptsHIcrr operon coding for phosphohistidine protein hexose phosphotransferase of the phosphotransferase system PTS (Journal of Biological Chemistry 262, 16241-16253 (1987)),
- the ptsI gene of the ptsHIcrr operon coding for enzyme I of the phosphotransferase system PTS (Journal of Biological Chemistry 262, 16241-16253 (1987)),
- the crr gene of the ptsHIcrr operon coding for the glucose-specific IIA component of the phosphotransferase system PTS (Journal of Biological Chemistry 262, 16241-16253 (1987)),
- the ptsG gene coding for the glucose-specific IIBC component (Journal of Biological Chemistry 261, 16398-16403 (1986)),
- the lrp gene coding for the regulator of the leucine regulon (Journal of Biological Chemistry 266, 10768-10774 (1991)),
- the csrA gene coding for the global regulator Csr (Journal of Bacteriology 175, 4744-4755 (1993)),
- the fadR gene coding for the regulator of the fad regulon (Nucleic Acids Research 16, 7995-8009 (1988)),
- the iclR gene coding for the regulator of the central intermediary metabolism (Journal of Bacteriology 172, 2642-2649 (1990)),
- the mopB gene coding for the 10 kd chaperone (Journal of Biological Chemistry 261, 12414-12419 (1986)), which is also known as groES,
- the ahpC gene of the ahpCF operon coding for the small subunit of alkyl hydroperoxide reductase (Proceedings of the National Academy of Sciences USA 92, 7617-7621 (1995)),
- the ahpF gene of the ahpCF operon coding for the large subunit of alkyl hydroperoxide reductase (Proceedings of the National Academy of Sciences USA 92, 7617-7621 (1995)),
- the cysK gene coding for cysteine synthase A (Journal of Bacteriology 170, 3150-3157 (1988)),
- the cysB gene coding for the regulator of the cys regulon (Journal of Biological Chemistry 262, 5999-6005 (1987)),
- the cysJ gene of the cysJIH operon coding for the flavoprotein of NADPH sulfite reductase (Journal of Biological Chemistry 264, 15796-15808 (1989), Journal of Biological Chemistry 264, 15726-15737 (1989)),
- the cysI gene of the cysJIH operon coding for the hemoprotein of NADPH sulfite reductase (Journal of Biological Chemistry 264, 15796-15808 (1989), Journal of Biological Chemistry 264, 15726-15737 (1989)), and
- the cysH gene of the cysJIH operon coding for adenylyl sulfate reductase (Journal of Biological Chemistry 264, 15796-15808 (1989), Journal of Biological Chemistry 264, 15726-15737 (1989)),
can be simultaneously overexpressed.

Furthermore, for the production of L-threonine, it can be advantageous not only to amplify the mglB gene but also to attenuate and, in particular, switch off one or more genes selected from the group comprising:
- the tdh gene coding for threonine dehydrogenase (Journal of Bacteriology 169, 4716-4721 (1987)),
- the mdh gene coding for malate dehydrogenase (E.C. 1.1.1.37) (Archives in Microbiology 149, 36-42 (1987)),
- the gene product of the open reading frame (orf) yjfA (Accession Number AAC77180 of the National Center for Biotechnology Information (NCBI, Bethesda, MD, USA)),
- the gene product of the open reading frame (orf) ytfP (Accession Number AAC77179 of the National Center for Biotechnology Information (NCBI, Bethesda, MD, USA)),
- the pckA gene coding for the enzyme phosphoenolpyruvate carboxykinase (Journal of Bacteriology 172, 7151-7156 (1990)),
- the poxB gene coding for pyruvate oxidase (Nucleic Acids Research 14 (13), 5449-5460 (1986)),
- the aceA gene coding for the enzyme isocitrate lyase (Journal of Bacteriology 170, 4528-4536 (1988)),
- the dgsA gene coding for the DgsA regulator of the phosphotransferase system (Bioscience, Biotechnology and Biochemistry 59, 256-251 [sic](1995)), which is also known as the mlc gene,
- the fruR gene coding for the fructose repressor (Molecular and General Genetics 226, 332-336 (1991)), which is also known as the cra gene, and
- the rpoS gene coding for the sigma³⁸ factor (WO 01/05939), which is also known as the katF gene.

In this context the term "attenuation" describes the decrease or switching-off of the intracellular activity, in a microorganism, of one or more enzymes (proteins) coded for by the appropriate DNA, for example by using a weak promoter or using a gene or allele which codes for an appropriate enzyme with a low activity or inactivates the appropriate enzyme (protein) or gene, and optionally combining these measures.

The attenuation measures generally reduce the activity or concentration of the appropriate protein to 0 to 75%, 0 to 50%, 0 to 25%, 0 to 10% or 0 to 5% of the activity or concentration of the wild-type protein or of the activity or concentration of the protein in the starting microorganism.

Furthermore, for the production of L-threonine it can be advantageous not only to amplify the mglB gene but also to switch off unwanted secondary reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms prepared according to the invention can be cultivated by the batch process, the fed batch process or the repeated fed batch process. A summary of known cultivation methods are [sic] provided in the textbook by Chmiel (Bioprozesstechnik 1. Einführung in die Bioverfahrenstechnik (Bioprocess Technology 1. Introduction to Bioengineering) (Gustav Fischer Verlag, Stuttgart, 1991)) or in the textbook by Storhas (Bioreaktoren und periphere Einrichtungen (Bioreactors and Peripheral Equipment) (Vieweg Verlag, Brunswick/Wiesbaden, 1994)).

The culture medium to be used must appropriately meet the demands of the particular strains. Descriptions of culture media for various microorganisms can be found in "Manual of Methods for General Bacteriology" of the American Society for Bacteriology (Washington DC, USA, 1981).

Carbon sources which can be used are sugars and carbohydrates, e.g. glucose, sucrose, lactose, fructose, maltose, molasses, starch and optionally cellulose, oils and fats, e.g. soya oil, sunflower oil, groundnut oil and coconut fat, fatty acids, e.g. palmitic acid, stearic acid and linoleic acid, alcohols, e.g. glycerol and ethanol, and organic acids, e.g. acetic acid. These substances can be used individually or as a mixture.

Nitrogen sources which can be used are organic nitrogen-containing compounds such as peptones, yeast extract, meat extract, malt extract, corn steep liquor, soya flour and urea, or inorganic compounds such as ammonium sulfate, ammonium chloride, ammonium phosphate, ammonium carbonate and ammonium nitrate. The nitrogen sources can be used individually or as a mixture.

Phosphorus sources which can be used are phosphoric acid, potassium dihydrogenphosphate or dipotassium hydrogenphosphate or the corresponding sodium salts. The culture medium must also contain metal salts, e.g. magnesium sulfate or iron sulfate, which are necessary for growth. Finally, essential growth-promoting substances such as amino acids and vitamins can be used in addition to the substances mentioned above. Suitable precursors can also be added to the culture medium. Said feed materials can be added to the culture all at once or fed in appropriately during cultivation.

The pH of the culture is controlled by the appropriate use of basic compounds such as sodium hydroxide, potassium hydroxide, ammonia or aqueous ammonia, or acid compounds such as phosphoric acid or sulfuric acid. Foaming can be controlled using antifoams such as fatty acid polyglycol esters. The stability of plasmids can be maintained by adding suitable selectively acting substances, e.g. antibiotics, to the medium. Aerobic conditions are maintained by introducing oxygen or oxygen-containing gaseous mixtures, e.g. air, into the culture. The temperature of the culture is normally 25°C to 45°C and preferably 30°C to 40°C. The culture is continued until the formation of L-amino acids or L-threonine has reached a maximum. This objective is normally achieved within 10 hours to 160 hours.

L-Amino acids can be analyzed by means of anion exchange chromatography followed by ninhydrin derivatization, as described by Spackman et al. (Analytical Chemistry 30, 1190-1206 (1958)), or by reversed phase HPLC, as described by Lindroth et al. (Analytical Chemistry 51, 1167-1174 (1979)).

The process according to the invention is used for the preparation of L-threonine by fermentation.

The present invention is illustrated in greater detail below with the aid of Examples.

The minimum medium (M9) and complete medium (LB) used for Escherichia coli are described by J.H. Miller (A Short Course in Bacterial Genetics (1992), Cold Spring Harbor Laboratory Press). The isolation of plasmid DNA from

Escherichia coli and all the techniques for restriction, ligation, Klenow treatment and alkaline phosphatase treatment are carried out according to Sambrook et al. (Molecular Cloning - A Laboratory Manual (1989), Cold Spring Harbor Laboratory Press). Unless described otherwise, the transformation of Escherichia coli is carried out according to Chung et al. (Proceedings of the National Academy of Sciences of the United States of America 86, 2172-2175 (1989)).

The incubation temperature in the preparation of strains and transformants is 37°C.

### Example 1

### Construction of expression plasmid pTrc99AmglB

The mglB gene from E. coli K12 is amplified using the polymerase chain reaction (PCR) and synthetic oligonucleotides. The nucleotide sequence of the mglB gene in E. coli K12 MG1655 (Accession Number AE000304, Blattner et al. (Science 277, 1453-1474 (1997)) is used as the starting material to synthesize PCR primers (MWG Biotech, Ebersberg, Germany). The sequences of the 5' ends of the primers are modified to create recognition sites for restriction enzymes. The recognition sequences for XbaI and HindIII, which are underlined in the nucleotide sequence shown below, are chosen for the mglB1 and mglB2 primers respectively:
mglB1: 5'-CCTGCTCTAGATAAACCGGAGATACCATG-3' (SEQ ID No. 1)
mglB2: 5'-CCGAAGCTTGTTGGCCATACAATAAGGGCG-3' (SEQ ID No. 2)

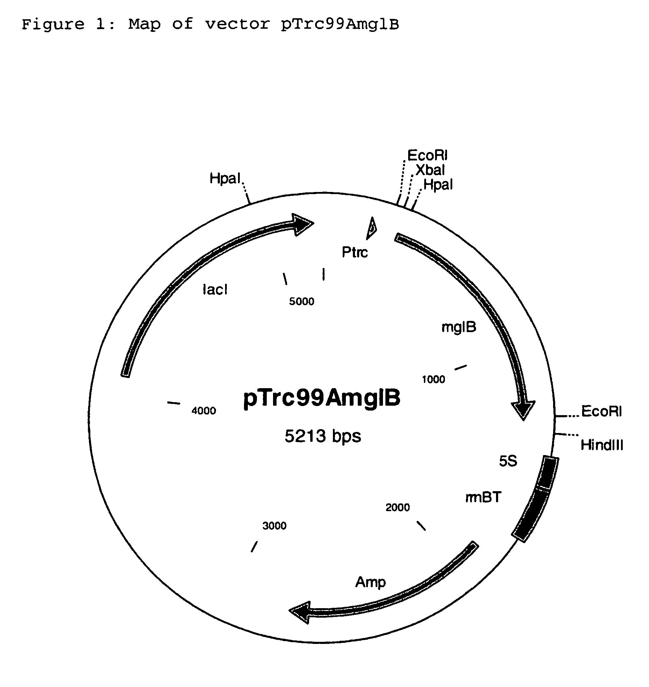

The chromosomal E. coli K12 MG1655 DNA used for the PCR is isolated with "Qiagen Genomic-tips 100/G" (QIAGEN, Hilden, Germany) in accordance with the manufacturer's instructions. An approx. 1100 bp DNA fragment can be amplified with the specific primers under standard PCR conditions (Innis et al. (1990) PCR Protocols. A Guide to Methods and Applications, Academic Press) using Pfu DNA polymerase (Promega Corporation, Madison, USA). The PCR product is cleaved with the restriction enzymes XbaI and HindIII and ligated with vector pTrc99A (Pharmacia Biotech, Uppsala, Sweden) that has been digested with the enzymes XbaI and HindIII. The E. coli strain XL1-Blue MRF' (Stratagene, La Jolla, USA) is transformed with the ligation mixture and plasmid-carrying cells are selected on LB agar supplemented with 50 µg/ml of ampicillin. The success of the cloning can be demonstrated, after isolation of the plasmid DNA, by control cleavage with the enzymes XbaI, HindIII, EcoRI and HpaI. The plasmid is called pTrc99AmglB (Figure 1).

### Example 2

### Preparation of L-threonine with the strain MG442/pTrc99AmglB

The L-threonine-producing E. coli strain MG442 is described in patent US-A-4,278,765 and is deposited in the Russian National Collection for Industrial Microorganisms (VKPM, Moscow, Russia) as CMIM B-1628.

The strain MG442 is transformed with expression plasmid pTrc99Amg1B, described in Example 1, and with vector pTrc99A and plasmid-carrying cells are selected on LB agar supplemented with 50 µg/ml of ampicillin. This procedure yields the strains MG442/pTrc99AmglB and MG442/pTrc99A. Chosen individual colonies are then multiplied further on minimum medium of the following composition: 3.5 g/l of Na₂HPO₄·2H₂O, 1.5 g/l of KH₂PO₄, 1 g/l of NH₄Cl, 0.1 g/l of MgSO₄·7H₂O, 2 g/l of glucose, 20 g/l of agar, 50 mg/l of ampicillin. The formation of L-threonine is verified in 10 ml batch cultures contained in 100 ml conical flasks. This is done by inoculating 10 ml of preculture medium of the following composition: 2 g/l of yeast extract, 10 g/l of (NH₄)₂SO₄, 1 g/l of KH₂PO₄, 0.5 g/l of MgSO₄·7H₂O, 15 g/l of CaCO₃, 20 g/l of glucose, 50 mg/l of ampicillin, and incubating for 16 hours at 37°C and 180 rpm on an ESR incubator from Kühner AG (Birsfelden, Switzerland). 250 µl of each of these precultures are transferred to 10 ml of production medium (25 g/l of (NH₄)₂SO₄, 2 g/l of KH₂PO₄, 1 g/l of MgSO₄·7H₂O, 0.03 g/l of FeSO₄·7H₂O, 0.018 g/l of MnSO₄·1H₂O, 30 g/l of CaCO₃, 20 g/l of glucose, 50 mg/l of ampicillin) and incubated for 48 hours at 37°C. The formation of L-threonine by the original strain MG442 is verified in the same way except that no ampicillin is added to the medium. After incubation the optical density (OD) of the culture suspension is determined using an LP2W photometer from Dr. Lange (Berlin, Germany) at a measurement wavelength of 660 nm.

The concentration of L-threonine formed is then determined in the sterile-filtered culture supernatant using an amino acid analyzer from Eppendorf-BioTronik (Hamburg, Germany) by means of ion exchange chromatography and postcolumn reaction with ninhydrin detection.

Table 1 shows the result of the experiment.

**Table 1**

| Strain | OD (660 nm) | L-threonine g/l |
|---|---|---|
| MG442 | 5.6 | 1.4 |
| MG442/pTrc99A | 3.8 | 1.3 |
| MG442/pTrc99AmglB | 5.2 | 2.0 |

### Brief Description of the Figure:

- Figure 1: Map of plasmid pTrc99AmglB containing the mglB gene

The indicated lengths are to be understood as approximate. The abbreviations and symbols used are defined as follows:
- Amp: ampicillin resistance gene
- lacI: gene for the repressor protein of the trc promoter
- Ptrc: trc promoter region, IPTG-inducible
- mglB: coding region of the mglB gene
- 5S: 5S rRNA region
- rrnBT: rRNA terminator region

The abbreviations for the restriction enzymes are defined as follows:
- EcoRI: restriction endonuclease from *Escherichia coli*
- HindIII: restriction endonuclease from *Haemophilus influenzae*
- HpaI: restriction endonuclease from *Haemophilus parainfluenzae*
- XbaI: restriction endonuclease from *Xanthomonas badrii*

## Claims

1. A process for the production of L-threonine, wherein the following steps are performed:
a) fermentation of modified microorganisms of the Enterobacteriaceae family which produce said L-amino acid,
b) concentration of said L-amino acid in the medium or in the cells of the microorganisms, and
c) isolation of said L-amino acid, wherein constituents of the fermentation broth and/or the biomass in its entirety or portions (> 0 to 100%) thereof optionally remain in the product,
wherein the modified microorganism compared to the unmodified microorganism comprises an over-expressed mglB gene or over-expressed nucleotide sequences coding for the gene product of said gene and wherein the mg1B gene, which codes for a periplasmatic galactose-binding transport protein, is from Enterobacteriaceae.

2. Process according to claim 1, wherein L-threonine is produced by fermenting microorganisms of the Enterobacteriaceae family in which in addition at the same time one or more of the genes chosen from the group consisting of:
2.1 the thrABC operon which codes for aspartate kinase, homoserine dehydrogenase, homoserine kinase and threonine synthase,
2.2 the pyc gene which codes for pyruvate carboxylase,
2.3 the pps gene which codes for phosphoenolpyruvate synthase,
2.4 the ppc gene which codes for phosphoenolpyruvate carboxylase,
2.5 the pntA and pntB genes which code for transhydrogenase,
2.6 the Escherichia coli rhtB gene coding for a protein imparting homoserine resistance,
2.7 the mqo gene which codes for malate:quinone oxidoreductase,
2.8 the Escherichia coli rhtC gene coding for a protein imparting threonine resistance,
2.9 the thrE gene which codes for the threonine export protein,
2.10 the gdhA gene which codes for glutamate dehydrogenase,
2.11 the hns gene which codes for the DNA-binding protein HLP-II,
2.12 the pgm gene, which codes for phosphoglucoisomerase,
2.13 the fba gene which codes for fructose bisphosphate aldolase,
2.14 the ptsH gene which codes for the phosphohistidine protein hexose phosphotransferase,
2.15 the ptsI gene which codes for enzyme I of the phosphotransferase system,
2.16. the crr gene which codes for the glucose-specific IIA component,
2.17 the ptsG gene which codes for the glucose-specific IIBC component,
2.18 the lrp gene, which codes for the regulator of the leucine regulon,
2.19 the csrA gene coding for the global regulator Csr
2.20 the fadR gene, which codes for the regulator of the fad regulon
2.21 the iclR gene, which codes for the regulator of the central intermediary metabolism,
2.22 the mopB gene, which codes for the 10 kD chaperone,
2.23 the ahpC gene, which codes for the small subunit of alkyl hydroperoxide reductase,
2.24 the ahpF gene, which codes for the large subunit of alkyl hydroperoxide reductase,
2.25 the cysK gene, which codes for cysteine synthase A,
2.26 the cysB gene, which codes for the regulator of the cys regulon,
2.27 the cysJ gene, which codes for the flavoprotein of NADPH-sulfite reductase,
2.28 the cysI gene, which codes for the haemoprotein of NADPH-sulfite reductase, and
2.29 the cysH gene, which codes for adenylyl sulfate reductase,
is or are over-expressed.

3. Process according to claim 1 or 2, wherein L-threonine is produced by fermenting microorganisms of the Enterobacteriaceae family in which in addition at the same time one or more of the genes chosen from the group consisting of:
3.1 the tdh gene which codes for threonine dehydrogenase,
3.2 the mdh gene which codes for malate dehydrogenase,
3.3 the gene product of the open reading frame (orf) yjfA of E. coli, when said microorganism is Escherichia coli,
3.4 the gene product of the open reading frame (orf) ytfP of E. coli, when said microorganism is Escherichia coli,
3.5 the pckA gene which codes for phosphoenolpyruvate carboxykinase,
3.6 the poxB gene which codes for pyruvate oxidase,
3.7 the aceA gene which codes for isocitrate lyase,
3.8 the dgsA gene which codes for the DgsA regulator of the phosphotransferase system,
3.9 the fruR gene which codes for the fructose repressor, and
3.10 the rpoS gene which codes for the sigma³⁸ factor, is or are switched off.

4. Recombinant microorganisms nf the Enterobacteriaceae family which produce L-threonine and wherein at least:
a) the thrABC operon which genes code for aspartate kinase, homoserine dehydrogenase, homoserine kinase and threonine synthase and
b) the mglB gene coding for the regulator of sigma-E-factor acitivity, or nucleotide sequences which code for the mglB gene product
are over-expressed.

5. Microorganisms according to claim 4, wherein the microorganisms originate from the genus Escherichia.

6. Microorganisms according to claim 5, wherein the microorganisms originate from the species E. coli.

## Patentansprüche

1. Verfahren zur Herstellung von L-Threonin, bei dem man folgende Schritte durchführt:
a) Fermentation von L-Threonin produzierenden modifizierten Mikroorganismen der Familie Enterobacteriaceae,
b) Anreicherung von L-Threonin im Medium oder in den Zellen der Mikroorganismen und
c) Isolierung des L-Threonins, wobei gegebenenfalls Bestandteile der Fermentationsbrühe und/oder die Biomasse in ihrer Gesamtheit oder Anteilen (> 0 bis 100%) davon im Produkt verbleiben,
wobei der modifizierte Mikroorganismus gegenüber dem unmodifizierten Mikroorganismus ein überexprimiertes mglB-Gen oder überexprimierte Nukleotidsequenzen umfaßt, die für das Genprodukt des genannten Gens kodieren und wobei das mglB-Gen, das für ein periplasmatisches, galaktosebindendes Transportprotein kodiert, aus der Familie Enterobacteriaceae stammt.

2. Verfahren nach Anspruch 1, bei dem man zur Herstellung von L-Threonin Mikroorganismen der Familie Enterobacteriaceae fermentiert, in denen man zusätzlich gleichzeitig eines oder mehrere der Gene, ausgewählt aus der Gruppe:
2.1 das für die Aspartatkinase, die Homoserin-Dehydrogenase, die Homoserinkinase und die Threoninsynthase kodierende thrABC-Operon,
2.2 das für die Pyruvat-Carboxylase kodierende pyc-Gen,
2.3 das für die Phosphoenolpyruvat-Synthase kodierende pps-Gen,
2.4 das für die Phosphoenolpyruvat-Carboxylase kodierende ppc-Gen,
2.5 die für die Transhydrogenase kodierenden Gene pntA und pntB,
2.6 das für ein Homoserinresistenz vermittelndes Protein kodierende Gen rhtB von Escherichia coli,
2.7 das für die Malat:Chinon Oxidoreduktase kodierende mqo-Gen,
2.8 das für ein Threoninresistenz vermittelndes Protein kodierende Gen rhtC von Escherichia coli,
2.9 das für das Threoninexportprotein kodierende thrE-Gen,
2.10 das für die Glutamat-Dehydrogenase kodierende gdhA-Gen,
2.11 das für das DNA-Bindeprotein HLP-II kodierende hns-Gen,
2.12 das für die Phosphoglukoisomerase kodierende pgm-Gen,
2.13 das für die Fructose-Bisphosphataldolase kodierende fba-Gen,
2.14 das für die Phosphohistidinproteinhexosephosphotransferase kodierende ptsH-Gen,
2.15 das für Enzym I des Phosphotransferase-Systems kodierende ptsI-Gen,
2.16 das für die glukosespezifische IIA-Komponente kodierende crr-Gen,
2.17 das für die glukosespezifische IIBC Komponente kodierende ptsG-Gen,
2.18 das für den Regulator des Leucin-Regulons kodierende lrp-Gen,
2.19 das für den globalen Regulator Csr kodierende csrA-Gen,
2.20 das für den Regulator des fad-Regulons kodierende fadR-Gen,
2.21 das für den Regulator des zentralen Intermediärstoffwechsels kodierende iclR-Gen,
2.22 das für das für das Chaperon 10 kD kodierende mopB-Gen,
2.23 das für die kleine Untereinheit der Alkylhydroperoxidreduktase kodierende ahpC-Gen,
2.24 das für die große Untereinheit der Alkylhydroperoxidreduktase kodierende ahpF-Gen,
2.25 das für die Cystein-Synthase A kodierende cysK-Gen,
2.26 das für den Regulator des cys-Regulons kodierende cysB-Gen,
2.27 das für das Flavoprotein der NADPH-Sulfitreduktase kodierende cysJ-Gen,
2.28 das für das Hämoprotein der NADPH-Sulfitreduktase kodierende cysI-Gen,
2.29 das für die Adenylylsulfat-Reduktase kodierende cysH-Gen,
überexprimiert.

3. Verfahren nach Anspruch 1 oder 2, bei dem man zur Herstellung von L-Threonin Mikroorganismen der Familie Enterobacteriaceae fermentiert, in denen man zusätzlich gleichzeitig eines oder mehrere der Gene, ausgewählt aus der Gruppe:
3.1 das für die Threonin-Dehydrogenase kodierende tdh-Gen,
3.2 das für die Malat-Dehydrogenase kodierende mdh-Gen,
3.3 falls es sich bei dem genannten Mikroorganismus um Escherichia coli handelt, das Genprodukt des offenen Leserahmens (orf) yjfA von E. coli,
3.4 falls es sich bei den genannten Mikroorganismus um Escherichia coli handelt, das Genprodukt des offenen Leserahmens (orf) ytfP von E. coli,
3.5 das für die Phosphoenolpyruvat-Carboxykinase kodierende pckA-Gen,
3.6 das für die Pyruvat-Oxidase kodierende poxB-Gen,
3.7 das für die Isocitrat-Lyase kodierende aceA-Gen,
3.8 das für den DgsA-Regulator des Phosphotransferase-Systems kodierende dgsA-Gen,
3.9 das für den Fructose-Repressor kodierende fruR-Gen,
3.10 das für den Faktor Sigma³⁸ kodierende rpoS-Gen,
ausschaltet.

4. Rekombinante L-Threonin produzierende Mikroorganismen der Familie Enterobacteriaceae, in denen zumindest:
a) das für die Aspartatkinase, die Homoserin-Dehydrogenase, die Homoserinkinase und die Threoninsynthase kodierende thrABC-Operon und
b) das für den Regulator der sigma-E-Faktor-Aktivität kodierende mglB-Gen oder für das mglB-Genprodukt kodierende Nukleotidsequenzen
überexprimiert sind.

5. Mikroorganismen nach Anspruch 4, bei denen die Mikroorganismen aus der Gattung Escherichia stammen.

6. Mikroorganismen nach Anspruch 5, bei denen die Mikroorganismen aus der Art E. coli stammen.

## Revendications

1. Procédé de production de L-thréonine, dans lequel on effectue les étapes suivantes :
a) la fermentation de micro-organismes modifiés de la famille des Enterobacteriaceae, qui produisent ledit acide L-aminé,
b) la concentration dudit acide L-aminé dans le milieu ou dans les cellules des micro-organismes, et
c) l'isolement dudit acide L-aminé, les constituants du bouillon de fermentation et/ou de la biomasse restant éventuellement dans leur intégralité ou sous forme de fractions (> 0 à 100 %) dans le produit,
dans lequel le micro-organisme modifié comprend, en comparaison du micro-organisme non modifié, un gène mglB surexprimé ou des séquences nucléotidiques surexprimées codant pour le produit génique dudit gène et dans lequel le gène mglB, qui code pour une protéine de transport périplasmique de liaison au galactose, provient d'une Enterobacteriaceae.

2. Procédé selon la revendication 1, dans lequel la L-thréonine est produite par fermentation de micro-organismes de la famille des Enterobacteriaceae, dans lesquels un ou plusieurs des gènes choisis dans le groupe suivant est ou sont aussi simultanément surexprimé(s) :
2.1 l'opéron thrABC, qui code pour l'aspartate kinase, l'homosérine déshydrogénase, l'homosérine kinase et la thréonine synthase,
2.2 le gène pyc, qui code pour la pyruvate carboxylase,
2.3 le gène pps, qui code pour la phosphoénolpyruvate synthase,
2.4 le gène ppc, qui code pour la phosphoénolpyruvate carboxylase,
2.5 les gènes pntA et pntB, qui codent pour une trans-hydrogénase,
2.6 le gène rhtB de l'espèce Escherichia coli, qui code pour une protéine conférant une résistance à l'homosérine,
2.7 le gène mqo, qui code pour la malate:quinone oxydoréductase,
2.8 le gène rhtC de l'espèce Escherichia coli, qui code pour une protéine conférant une résistance à la thréonine,
2.9 le gène thrE, qui code pour la protéine d'export de la thréonine,
2.10 le gène gdhA, qui code pour la glutamate déshydrogénase,
2.11 le gène hns, qui code pour la protéine de liaison à l'ADN HLP-II,
2.12 le gène pgm, qui code pour une phosphogluco-isomérase,
2.13 le gène fba, qui code pour la fructose-biphosphate aldolase,
2.14 le gène ptsH, qui code pour la phosphohistidino-protéine-hexose phosphotransférase,
2.15 le gène ptsI, qui code pour l'enzyme I du système de phosphotransférase,
2.16 le gène crr, qui code pour le composant IIA spécifique au glucose,
2.17 le gène ptsG, qui code pour le composant IIBC spécifique au glucose,
2.18 le gène lrp, qui code pour le régulateur du régulon de la leucine,
2.19 le gène csrA codant pour le régulateur global Csr,
2.20 le gène fadR, qui code pour le régulateur du régulon fad,
2.21 le gène iclR, qui code pour le régulateur du métabolisme central intermédiaire,
2.22 le gène mopB, qui code pour la molécule chaperonne de 10 kD,
2.23 le gène ahpC, qui code pour la petite sous-unité de l'alkylhydroperoxyde réductase,
2.24 le gène ahpF, qui code pour la grosse sous-unité de l'alkylhydroperoxyde réductase,
2.25 le gène cysK, qui code pour la cystéine synthase A,
2.26 le gène cysB, qui code pour le régulateur du régulon de la cys,
2.27 le gène cysJ, qui code pour la flavoprotéine de la NADPH-sulfite réductase,
2.28 le gène cysI, qui code pour l'hémoprotéine de la NADPH-sulfite réductase,
2.29 le gène cysH, qui code pour l'adénylylsulfate réductase,

3. Procédé selon la revendication 1 ou 2, dans lequel la L-thréonine est produite par fermentation de micro-organismes de la famille des Enterobacteriaceae, dans lesquels un ou plusieurs des gènes choisis dans le groupe suivant est ou sont aussi simultanément éteint(s) :
3.1 le gène tdh, qui code pour la thréonine déshydrogénase,
3.2 le gène mdh, qui code pour la malate déshydrogénase,
3.3 le produit du gène yjfA du cadre de lecture ouvert (orf) de l'espèce E. coli, lorsque ledit micro-organisme est représenté par l'espèce Escherichia coli,
3.4 le produit du gène ytfP du cadre de lecture ouvert (orf) de l'espèce E. coli, lorsque ledit micro-organisme est représenté par l'espèce Escherichia coli,
3.5 le gène pckA, qui code pour la phosphoénolpyruvate carboxykinase,
3.6 le gène poxB, qui code pour la pyruvate oxydase,
3.7 le gène aceA, qui code pour l'isocitrate lyase,
3.8 le gène dgsA, qui code pour le régulateur DgsA du système de la phosphotransférase,
3.9 le gène fruR, qui code pour le répresseur du fructose, et
3.10 le gène rpoS, qui code pour le facteur sigma³⁸.

4. Micro-organismes recoubinants de la famille des Enterobacteriaceae, qui produisent de la L-thréonine et dans lesquels sont surexprimés au moins :
a) l'opéron thrABC, dont les gènes codent pour l'aspartate kinase, l'homosérine déshydrogénase, l'homosérine kinase et la thréonine synthase, et
b) le gène mglB codant pour le régulateur de l'activité du facteur sigma E, ou les séquences nucléotidiques qui codent pour le produit du gène mglB.

5. Micro-organismes selon la revendication 4, dans lesquels les micro-organismes sont issus du genre Escherichia.

6. Micro-organismes selon la revendication 5, dans lesquels les micro-organismes sont issus de l'espèce E. coli.
